# EUROPÄISCHE PATENTSCHRIFT

(11) **EP 2 142 271 B1**
(45) Veröffentlichungstag und Bekanntmachung des Hinweises auf die Patenterteilung: **05.10.2016**
(21) Anmeldenummer: 08749051.2
(22) Anmeldetag: 23.04.2008
(51) Int. Cl.: B01D 11/04, G01N 33/543, B03C 1/28

(54) **VERFAHREN ZUR MAGNETISCH UNTERSTÜTZTEN EXTRAKTION**
METHOD FOR MAGNETICALLY SUPPORTED EXTRACTION
PROCÉDÉ D'EXTRACTION À ASSISTANCE MAGNÉTIQUE

(30) Priorität: 28.04.2007 DE 102007020220
(43) Veröffentlichungstag der Anmeldung: 13.01.2010
(73) Patentinhaber: Karlsruher Institut für Technologie, 76131 Karlsruhe (DE)
(72) Erfinder: FRANZREB, Matthias, 76185 Karlsruhe (DE); BECKER, Jörg, 76135 Karlsruhe (DE)
(86) Internationale Anmeldenummer: PCT/EP2008/003245
(87) Internationale Veröffentlichungsnummer: WO 2008/131891

(56) Entgegenhaltungen:
- WO-A-03/089906
- SAITOH T ET AL: "USE OF SURFACTANT-MEDIATED PHASE SEPARATION (CLOUD POINT EXTRACTION) WITH AFFINITY LIGANDS FOR THE EXTRACTION OF HYDROPHILIC PROTEINS" TALANTA, ELMSFORD,NY, US, Bd. 42, Nr. 1, 1. Januar 1995 (1995-01-01), Seiten 119-127, XP000603770
- SUZUKI M, KAMIHIRA M, SHIRAISHI T, TAKEUCHI H, KOBAYASHI T: "AFFINITY PARTITIONING OF PROTEIN-A USING A MAGNETIC AQUEOUS 2-PHASE SYSTEM" JOURNAL OF FERMENTATION AND BIOENGINEERING, Bd. 80, Nr. 1, 1995, Seiten 78-84, XP002490533 in der Anmeldung erwähnt
- S. FLYGARE, P. WIKSTR6M, G. JOHANSSON AND P.-O. LARSSON: "Magnetic aqueous two-phase separation in preparative applications" ENZYME MICROB. TECHNOL., Bd. 12, Februar 1990 (1990-02), Seiten 95-103, XP002490534

## Beschreibung

Die Erfindung betrifft ein Verfahren zur magnetisch unterstützen Extraktion gemäß des ersten Patentanspruchs.

Eine Extraktion der eingangs genannten Art ist ein physikalisches Stofftrennverfahren, bei dem mit Hilfe eines Extraktionsmittels eine Komponente aus einem Stoffgemisch herausgelöst und separiert wird. Das Extraktionsmittel wird dann nach der Separierung wieder entfernt. Extraktionen finden ihre besondere Bedeutung in der Aufbereitung biologischer und biochemischer Substanzen, und zwar sowohl zur Gewinnung als auch zur Aufreinigung dieser Substanzen.

Hierfür beispielhaft offenbaren [1-5] Verfahren zur Extraktion von Vitamin A und D aus Fischölen oder die Extraktion von Benzylpenizillin aus einer zuvor filtrierten Fermentationsbrühe. Aufgrund der Sensitivität komplexerer Biomoleküle gegenüber unpolaren Lösungsmittel wurden sog. **ATPS**-Verfahren (**ATPS = Aqueous Two Phase Systems**) entwickelt (vgl. **[3-5]**). **ATPS** sind wässrige Lösungen verschiedener hydrophiler Polymere (z.B. Polyetyhlenglykol (PEG), Dextran) oder von einem Polymer und anorganischen Salzen (z.B. Ammoniumsulfat, Kaliumphosphat). Oberhalb kritischer Konzentrationen dieser Komponenten setzt eine Trennung der Lösung in zwei, je mit einer der Komponenten angereicherten, Phasen ein, in denen Proteine ohne Denaturierung löslich sind **[2].** Wird nun eine Biosuspension in ein **ATPS** überführt, reichern sich ihre Bestandteile entsprechend der jeweiligen Verteilungskoeffizienten in einer der beiden Phasen an. So finden sich im Falle eines PEG-Dextran Systems die Zelltrümmer eines Fermentationshomogenisats in der Regel überwiegend in der Dextranphase während die Proteine überwiegend in die wässrige PEG-Phase übergehen.

Ein Problem der **ATPS** sind dabei häufig unzureichende Verteilungskoeffizienten, die eine zeit- und apparatetechnisch aufwendige mehrstufige Verfahrensweise notwendig machen. Um die Selektivität dieses Trennverfahrens zu steigern, werden daher die phasenbildenden Polymere z.B. mit affinen Liganden gezielt funktionalisiert. Damit wirken diese spezifisch auf ein bestimmtes Zielmolekül bzw. eine Gruppe von Zielmolekülen ein und binden diese selektiv an sich an. Ein weiteres grundsätzliches Problem ist die aufgrund des wässrigen Charakters beider Phasen sehr geringe Oberflächenspannung an den Phasengrenzflächen. Folglich besteht nur ein geringes Bestreben des Systems zur Verringerung der Grenzflächen und damit zur Koaleszenz, d.h. die durch Vermischung gebildeten Emulsionen entmischen sich nur sehr langsam. Als Ausweg werden oftmals Zentrifugationsprozesse eingesetzt, die jedoch apparativ aufwendig und damit kostenintensiv sind und zudem in ihrem Durchsatz begrenzt sind. Außerdem entstehen bei einer Trennung insbesondere im Rahmen einer großtechnischen industriellen Nutzung zunächst große Mengen stark polymerhaltiger bzw. stark salzhaltiger Abwässer. Neben den resultierenden Chemikalienkosten spielen folglich auch die Kosten für die Abwasserbehandlung eine Rolle. Folglich strebt man die Umsetzung von **ATPS**-Verfahren an, die ein Recycling mindestens einer der beteiligten Phasen ermöglichen.

In [**6-8**] wird eine Beschleunigung der Phasentrennung von **ATPS** unter der Zugabe magnetischer Partikel beschrieben. Dabei kamen jedoch stets unfunktionalisierte magnetische Nano- oder Mikropartikel zum Einsatz, so dass die Verteilungskoeffizienten des Systems durch die Beimischung nicht verbessert wurden.

Folglich kamen in einer späteren Arbeit **[9]** funktionelle magnetische Partikel in Kombination mit dem **ATPS**-Verfahren zum Einsatz. Dabei wurden Versuche zur Ermittlung des Verteilungskoeffizienten von Immunglobulinen unter Zugabe von mit Protein A funktionellisierten Magnetpartikeln im kleinen Batch-Maßstab (ca. 1 mL) durchgeführt. Dabei kam ein PEG-Phosphat (**ATPS**-System) zum Einsatz, wobei der scheinbare Verteilungskoeffizient der PEG-reichen Phase durch die Zugabe der Magnetpartikel je nach Versuchsdurchführung um den Faktor 4-35 gesteigert werden konnte. Der Ansatz verwendet jedoch ein **ATPS**-System der vorgenannten Art ohne grundsätzliche Änderung, sodass bei einer großtechnischen Umsetzung des Verfahrens nach wie vor mit einer enormen Salzfracht im entstehenden Abwasser zu rechnen ist. Zudem ist die im Falle der Verwendung eines realen Zellhomogenisats resultierende Reinheit nicht besonders hoch, da die PEG-reiche Phase neben der gewünschten Substanz auch zahlreiche andere Proteine an sich bindet.

Folglich gewinnt beim Versuch eines vollständigen Recyclings der vorgenannten ATPS der Einsatz einer sog. **Cloud-Point Extraction (CPE oder CP-ATPS)** an Bedeutung. Im Falle der **CP-ATPS** kommt ein in die Lösung einzumischendes **Phase Forming Polymer** (Phasenformendes Polymer, **PFP**), d.h. ein Tensid zum Einsatz. Dieses bewirkt bei Über- oder Unterschreitung einer kritischen Temperatur (Trübungspunkt) oder einer anderen kritischen physikalischen Größe ein Aufspalten einer zunächst homogenen Lösung in zwei makroskopische Phasen, wobei sich das **PFP** (Tensid) sich nahezu ausschließlich in einer der Phasen anreichert.

Die Vorteile der **CP-ATPS** gegenüber klassischen **ATPS** liegen einerseits in der Zurückgewinnbarkeit der zur Bildung des **ATPS** herangezogenen Substanz. Andrerseits ermöglichen entsprechende Systeme eine saubere Abtrennung von z.B. Zelltrümmern, da durch die sehr niedrige Grenzflächenspannung zwischen den Phasen die Tendenz zur Feststoffakkumulation an der Phasengrenze nur gering ist. Eine selbst für **ATPS** geringe Grenzflächenspannung sowie der geringe Dichteunterschied der zu trennenden Phasen bedingt jedoch eine noch schwächere Koaleszenztendenz und sehr langsame und oftmals unvollständige Phasentrennung.

Davon ausgehend liegt die **Aufgabe der Erfindung** darin, ein auch im industriellen großtechnischen Maßstab einsetzbares **CP-ATPS** Verfahren zur magnetisch unterstützen Extraktion vorzuschlagen, das eine effiziente Abtrennung und Vorreinigung von Biomolekülen auch aus fest-stoffhaltigen Medien erlaubt und insbesondere eine signifikant verbesserte Rezyklierbarkeit der Systemkomponenten bei gleichzeitig verbesserter selektiven Abtrennungsleistung in nur einem Verfahrensschritt ermöglicht.

Die Aufgabe wird durch ein Verfahren mit den Merkmalen des Anspruchs 1 gelöst. Auf diesen rückbezogene Unteransprüche geben vorteilhafte Ausgestaltungen des Verfahrens wieder.

Die Aufgabe wird mit einem Verfahren zur magnetisch unterstützen Extraktion einer Zielsubstanz aus einer wässrigen Lösung mit mehreren Substanzen, dem sog. **SMEP**-Verfahren **(SMEP = Smart Magnetic Extraction Phases)** gelöst. Bei diesem Verfahren erfolgt ein Einmischen von funktionellen magnetischen Partikeln mit einer Affinität zu der gewünschten Zielsubstanz in die wässrige Lösung mit der zu extrahierenden Zielsubstanz (Ausgangslösung), wobei die Zielsubstanz sich an die Partikel vorzugsweise chemisch oder adhäsiv anbindet. Wesentlich ist auch eine vorzugsweise darauf folgende Eingabe eines **PFP**, vorzugsweise eines Tensids in die wässrige Lösung mit den magnetischen Partikeln, wobei die entstehende Lösung einen Trübungspunkt (**CP**-Punkt, **CP = Cloud-Point)** aufweist.

Wird der Trübungspunkt, d.h. der CP-Punkt überschritten, erfolgt je nach Richtung der Überschreitung eine Überführung der Lösung oder Teile dieser von einem einphasigen in einen zweiphasigen Zustand oder umgekehrt. Der Trübungspunkt wird üblicherweise durch eine bestimmte Temperatur, der Trübungstemperatur wiedergegeben, wobei sich in der Regel bei Temperaturen unterhalb dieser der einphasige Zustand und oberhalb dieser der zweiphasige (bei disperser Durchmischung trübe) Zustand einstellt. Der Trübungspunkt wird wesentlich von der Zusammensetzung der Lösung, insbesondere der Konzentration des **PFP** oder Tensids in der Lösung beeinflusst.

Es liegt im Rahmen der Erfindung, wenn der Trübungspunkt auch durch andere Größen bestimmt wird. Anstelle oder in Kombination eines Temperaturwerts ist der Trübungspunkt auch durch einen pH-Wert, eine Ionenstärke oder einer Kombination aus mehreren Größen definierbar, wobei die vorgenannte Phasenänderung durch eine Änderung des pH-Wertes oder auch der Ionenstärke steuerbar ist.

Im vorliegenden Verfahrensschritt bildet sich bei der vorgenanten Eingabe des **PFP** oder Tensids zunächst eine einphasige wässrige Tensidlösung (oder **PFP**-Lösung, im Folgenden pauschal Tensidlösung genannt) aus, die anschließend nach optionalen zusätzlichen Homogenisierungsschritten in vorgenannter Weise in einen zweiphasigen Zustand überführt wird. Dabei entsteht beginnend an den Partikeln mit der angebundenen Zielsubstanz eine erste tensidhaltige (bzw. (PFP-haltige) disperse Phase in einer dieser umgebenden zweiten Phase. Dabei weist die tensidhaltige Phase (**PFP**-haltige Phase) im Vergleich zur zweiten Phase ein geringeres Aufnahmevermögen für alle nicht an die Partikel gebundenen oder zumindest die von der Zielsubstanz zu separierenden Substanzen auf. Lediglich die zu separierende Zielsubstanz wird durch die funktionellen Magnetpartikel gebunden und somit mit diesen in der tensidhaltigen Phase (**PFP**-haltige Phase) gehalten.

In einem weiteren Verfahrensschritt erfolgt eine Separierung der Phasen im Zweiphasengebiet durch Einwirken eines Magnetfeldes. Magnetische Kräfte wirken selektiv auf die magnetischen Partikel ein und ziehen diese gemeinsam mit der angebundenen Zielsubstanz und der tensid- oder **PFP**-haltigen Phase in Richtung höherer magnetischer Feldstärken. Die koaleszierte tensidhaltige Phase ist dann an dieser Stelle gemeinsam mit den magnetischen Partikel und der Zielsubstanz für eine weitere Verarbeitung von der zweiten Phase beispielsweise durch Absaugung abtrennbar.

Die Verwendung eines Tensids (oder eines **PFP**) mit den genannten Eigenschaften (weitgehender Ausschluss aller relevanten Substanzen) wird erst in Kombination mit funktionellen magnetischen Partikeln sinnvoll, bietet dann aber verschiedene Vorteile. Ohne die Verwendung von Nano- oder Mikropartikeln, die die Zielsubstanz binden und im gebundenen Zustand in die disperse Phase überführen, wäre dagegen der Einsatz eines **ATPS** bei dem eine Phase alle Biomoleküle praktisch ausschließt für die Bioseparation nutzlos, da keine Stofftrennung stattfindet. Umgekehrt ist aber auch der Einsatz der Nano- und Mikropartikel zusammen mit einem ATPS dessen disperse Phase Biomoleküle praktisch ausschließt nur in Kombination mit einem **CP-ATPS** besonders vorteilhaft, das reversibel zwischen einem einphasigen und einen zweiphasigen Zustand durch die Veränderung eines Parameters geschaltet werden kann. Im Falle eines herkömmlichen **ATPS,** das ständig im zweiphasigen Zustand vorliegt, würden die Partikel dagegen permanent von der polymerreichen, die Zielsubstanz ausschließenden, Phase umhüllt.

Eine Anbindung der Zielsubstanz an die Partikel und damit die angestrebte Stofftrennung wäre daher nicht möglich.

Die Erfindung wird anhand von Versuchsbeispielen mit den folgenden Figuren näher erläutert, wobei der Begriff Tensid grundsätzlich eine alternative Verwendung von **PFP** mit einschließt. Es zeigen
**Fig.1** das Grundablaufschema eines **SMEP**-Verfahrensbeispiels,
**Fig.2** ein Ablaufschema eines **SMEP**-Verfahrensbeispiels mit einer getrennten Rückführung der funktionellen Magnetpartikel (**FMP**) und des Tensids sowie
**Fig.3** ein Ablaufschema des **SMEP**-Verfahrens gemäß **Fig.2**, jedoch mit einer zeitversetzten separaten Einspeisung der rückgeführten funktionellen Magnetpartikel und der Tenside.

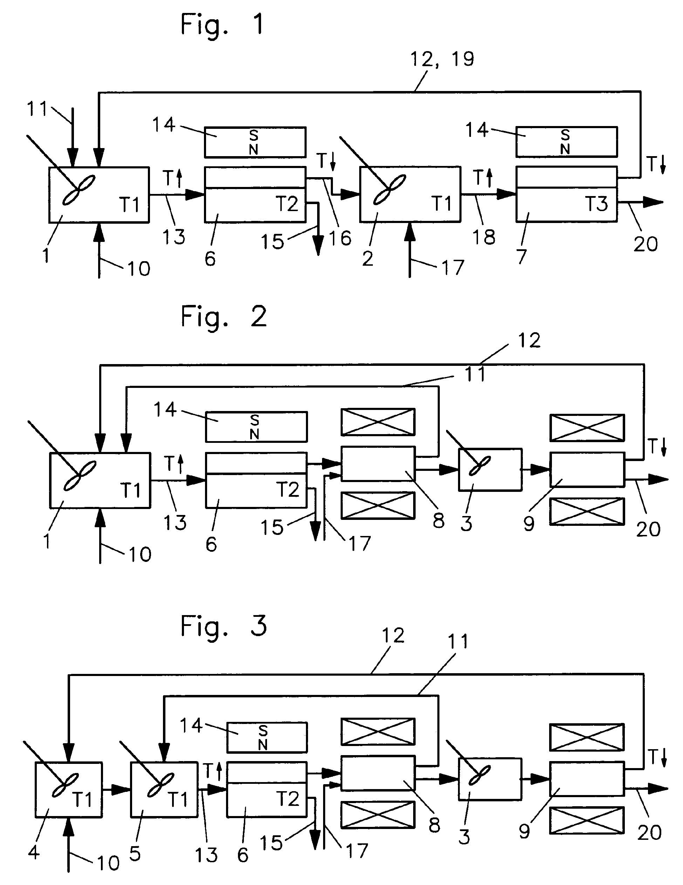

Bei allen in den **Fig.1 bis 3** dargestellten Verfahrensbeispielen wird eine Kaskade von mehreren Mischgefäßen **1 bis 5** und magnetischen Separatoren **6 bis 9** durchfahren, wobei zunächst das Ausgangsmedium **10** (wässrige Lösung), das eine gewünschte Zielsubstanz, beispielsweise ein Biomolekül (z.B. ein Protein) neben einer Vielzahl unerwünschter Begleitstoffe enthält, mit den funktionellen Magnetpartikel **12** und mit dem Tensid **11** zu einem Lösungsgemisch **13** zusammengebracht wird, die Lösung mittels eines Parameterwechsels, in den Beispielen nur die Temperatur (**T1** und **T2**), zwischen einem einphasigen und einem mehrphasigen Zustand hin- und hergeschaltet wird und nach Phasentrennung die getrennten Phasen als separate Stoffströme entnehmbar sind. Die Temperatur **T1** liegt im Beispiel dabei unterhalb, die Temperatur **T2** dabei oberhalb des Trübungspunktes (**CP**-Wert).

Die funktionellen Magnetpartikel 12 kommen in der Lösung mit der Zielsubstanz in Kontakt und binden diese selektiv und mit hoher Affinität. Als Funktionalisierung der funktionellen Magnetpartikel können dabei alle in der Bioproduktaufarbeitung bekannten Gruppen benutzt werden, wie z.B. ionenaustauschaktive Moleküle, Moleküle mit hydrophober Wechselwirkung, gruppenspezifische Liganden wie Metallchelatbildner und Protein A oder spezifische Antikörper.

**Fig.1** zeigt das Grundschema eines "Smart Magnetic Extraction Process" (**SMEP**-Verfahren). Die wässrige Ausgangslösung **10** wird gemeinsam mit funktionellen Magnetpartikeln **12** und mit dem Tensid **11** in einer ersten Mischkammer **1** eingegeben und dort auf eine Temperatur **T1** temperiert, wobei sich eine einphasige wässrige Tensidlösung bildet. Die funktionellen magnetischen Partikel weisen dabei eine Affinität zu der zu separierenden Zielsubstanz auf und binden diese an sich.

Das Einmischen der funktionellen Magnetpartikel **12** und des Tensid **11** in die Ausgangslösung erfolgt im Rahmen der Erfindung zeitgleich (parallel) oder hintereinander (seriell). Ein Einmischen zunächst der Magnetpartikel und anschließenden Einmischen des Tensids führt jedoch grundsätzlich zu einer schnellere Anbindung der Zielsubstanz an die magnetischen Partikel, da vor einmischen des Tensids eine Behinderung der Anbindung der Zielsubstanz an die Partikel durch das die Zielsubstanz verdrängende Tensid ausgeschlossen wird, insbesondere wenn das Tensid sich selbst an den Partikeln anlagert.

Die einphasige wässrige Tensidlösung wird anschließend als Lösungsgemisch **13** aus der ersten Mischkammer **1** entnommen, auf die Temperatur **T2** oberhalb des Trübungspunktes erwärmt und auf diese Weise in einen zweiphasigen Zustand überführt in einen ersten magnetischen Separator **6** eingeleitet.

Der zum Einstellen des ein- oder zweiphasigen Zustands des **ATPS** benutzte Parameter ist in diesem Beispiel die Temperatur, wobei auch andere Betriebsparameter wie z.B. Ionenstärke oder pH-Wert denkbar sind.

Die mit der Zielsubstanz beladenen funktionellen Magnetpartikel gehen dabei nach Voraussetzung quantitativ in die tensidreiche (polymerreiche), disperse Phase über. Ungebundene Biomoleküle sowie Fest-Stoffe wie z.B. Zelltrümmer verbleiben dagegen in der homogenen, an Tensiden (bzw. **PFP**) abgereicherten zweiten Phase. Aufgrund der magnetischen Eigenschaften der funktionellen Magnetpartikel lässt sich die nach der Temperaturerhöhung einsetzende Phasentrennung durch ein überlagertes und auf die magnetischen Partikel einwirkendes Magnetfeld (illustriert durch einen Permanentmagneten) in dem ersten magnetischen Separator **6** mit Permanentmagneten **14** oder gleich wirkenden Magnetfeldquellen stark beschleunigen.

Nach Abschluss der Phasentrennung wird die die Verunreinigungen enthaltende Phase als Abwasserstrom **15** abgeführt und die die Partikel enthaltende tensidhaltige Phase **16** wieder unter den Trübungspunkt auf die Temperatur **T1** abgekühlt und einer zweiten Mischkammer **2** zugeführt.

In der zweiten Mischkammer **2** erfolgt eine Einmischung einer Substanz in die einphasig vorliegende Phase, die als wässriger Elutionspuffer **17** eine Ablösung der gebundenen Zielsubstanz von den funktionellen Magnetpartikeln bewirkt. Am Ende dieses Verfahrensschritts verlässt die Zielsubstanz im Gemisch **18** die zweite Mischkammer in ungebundener gelöster Form. Danach wird das Gemisch **18** erneut auf eine Temperatur **T3** oberhalb des Trübungspunktes des Gemischs aufgewärmt und in einen zweiten magnetischen Separator **7** mit Permanentmagenten **14** gleitet.

Das in den zweiten magnetischen Separator **7** eingeleitete Gemisch liegt somit in einem zweiphasigen Zustand vor, wobei eine Phase die Zielsubstanz und die andere Phase die magnetischen Partikel beinhaltet. Die funktionellen Magnetpartikel gehen dabei wieder in die polymerreiche, disperse Phase über. Da die Zielsubstanz nun aber in gelöster freier Form vorliegt, wird sie aus der polymerreichen Phase verdrängt und reichert sich komplett in der an Polymer abgereicherten Phase an. Nach einer wiederum durch ein überlagertes Magnetfeld beschleunigten Phasentrennung, kann das Zielmolekül als Produkt über die polymerarme Phase abgezogen werden. Das Produkt liegt dabei in feststofffreier, aufgereinigter Form vor. Während die Phase mit den funktionellen magnetischen Partikeln **12** als Partikelrezyklierung **19** der ersten Mischkammer zugeführt wird, erfolgt eine Ableitung der Phase mit der gelösten isolierten Zielsubstanzlösung 20 zu einer nicht weiter dargestellten Verwendung.

Die eluierten funktionellen Magnetpartikel sowie der Großteil des Tensids können grundsätzlich innerhalb des Prozesses rezykliert werden. Im Idealfall wird auch das Elutionsmittel aus der polymerreichen Phase ausgeschlossen und eine direkte Rückführung ist möglich.

**Fig.2** repräsentiert dagegen ein Verfahrensbeispiel, das sich einerseits durch eine getrennte Rückführung der funktionellen Magnetpartikel (**FMP**) und des Tensids in die erste Mischkammer 1 kennzeichnet, andererseits zur Abtrennung der funktionellen Magnetpartikel 12 aus einer homogenen Phase jeweils auf Hochgradientenmagnetseparatoren **8, 9** zurückgreift.

Dabei wird in dem ersten Hochgradientenmagnetseparator **8** zunächst das Tensid **11** von den Partikeln getrennt und in das erste Mischkammer **1** rezykliert. Die zurückgehaltenen Partikel mit der Zielsubstanz werden in die Mischkammer **3** weitergeleitet. Vorzugsweise wird der erste Hochgradientenmagnetseparator dabei zyklisch betrieben, d.h. in einem ersten Teilschritt erfolgt die Abspaltung des Tensids, während in einem zweiten Teilschritt die im Hochgradientenmagentseparator festgehaltenen Partikel mit einen Elutionspuffer **17** aus diesen herausgespült werden.

Der Elutionspuffer gelangt mit den Partikeln anschließend in die dritte Mischkammer **3**, indem die eigentliche Lösung der Zielsubstanz von den Partikeln erfolgt. Die Abtrennung der Magnetpartikel erfolgt dann anschließend in einem zweiten Hochgradientenmagnetseparator **9**.

Die rezyklierten funktionellen Magnetpartikel **12** und die rezyklierten Tenside 11 werden jeweils in das erste Rührgefäß eingeleitet, d.h. es erfolgt eine gemeinsame Einmischung dieser in die wässrige Lösung mit der Zielsubstanz (Ausgangslösung **10**).

Wie im vorgenannten und in **Fig.1** dargestellten Verfahrensbeispiel erfolgt die Einmischung mit einer Temperatur **T1** unterhalb des Trübungspunkts der entstandenen einphasigen wässrigen Tensidlösung. Ebenso wird diese über den Trübungspunkt auf **T2** aufgewärmt, d.h. im zweiphasigen Zustand in einen ersten magnetischen Separator **6** eingeleitet und dort unter Einwirkung eines Magnetfeldes (Permanentmagnet **14** oder gleich wirkende Magnetquelle) in einen Abwasserstrom **15** und der tensidhaltigen zweiten Phase **16** aufgeteilt.

Abweichend von dem erstgenannten Versuchsbeispiel (vgl. **Fig.1**) wird die tensidhaltige zweite Phase **16** erst nach einer Abtrennung der in dieser enthaltenen in Lösung befindlichen Tenside mit einem wässrigen Elutionspuffer **17** vermischt. Die Abrennung erfolgt vorzugsweise vor einer erneuten Abkühlung der partikel- und tensidhaltigen Phase in einem ersten Hochgradientenmagnetseparator **8**. Während die darin abgetrennten Tenside direkt in die erste Mischkammer rzykliert werden, werden die magnetischen Partikel mit der Zielsubstanz sowie ein Elutionspuffer **17** in eine dritten Mischkammer 3 eingeleitet.

Schließlich ist auch der Fall denkbar, dass die eingesetzten Tenside im Einphasengebiet einen negativen Einfluss auf die Anbindung der Zielmoleküle an die funktionellen Magnetpartikel ausüben. In diesem Fall ist eine Abwandlung des Versuchsbeispiels gemäß **Fig.2** vorteilhaft, in der die getrennte Rückführung und Einmischung der Tenside **11** (**PFP**) und der funktionellen Magnetpartikel **12** (**FMP**) auch im Bindeschritt genutzt wird (vgl. **Fig.3**). Dabei wird in einem ersten Schritt die Ausgangslösung **10** in einer vierten Mischkammer **4** zunächst nur mit den funktionellen Magnetpartikeln **12** zu einer wässrigen Lösung vermischt, so dass die Bindung ungestört von möglichen Einflüssen der Tenside stattfinden kann. Anschließend erfolgt nach Weiterleitung der wässrigen Lösung in eine fünfte Mischkammer 5 in dieser die Zugabe der Tenside **11** (bzw. **PFP**). Der weitere Prozessverlauf entspricht der in **Fig.2** dargestellten Abfolge.

### Literatur:

[1] Ullmanns Encyclopedia of Technical Chemistry, Vol. B3, Chapter 6: Liquid-Liquid Extraction,
[2] Ullmanns Encyclopedia of Technical Chemistry, Vol. B3, Chapter 11: Biochemical Separations,
[3] Albertsson, P .A.: Partition of Cell Particles and Macromolecules, New York: Wiley (1986)
[4] Johansson, G.: Aqueous two-phase systems in protein purification, J. Biotech. 3 (1985) 11-18
[5] Scopes, R.K.: Protein purification in the nineties; Biotechnol. Appl, Biochem., 23 (1996) 197-204
[6] Wikström, P. et al.: Magnetic aqueous two-phase separation: A new technique to increase rate of phase-separation, using dextran-ferrofluidor larger iron oxide particles. Anal. Biochem. (1987) 167: 331-339.
[7] Wikström, P. et al.: Magnetically enhanced aqueous two-phase separation, in Separation using aqueous phase systems, D. Fisher and I.A. Sutherland, Editors. (1989) Plenum Publishing Corp. 455-461
[8] Flygare, S. et al.: Magnetic aqueous two-phase Separation in preperative applications, Enzyme Microb. Tech., 12 (1990) 95-103
[9] Suzuki, M. et al.: Affinity Partitioning of Protein A using a Magnetic Aqueous Two-Phase System, J. Ferment. Bioeng. 80 (1995) 78-84
[10] Franzreb, M. et al.: Product recovery by high-gradient magnetic fishing (HGMF), in PROCESS SCALE BIOSEPARATIONS FOR THE BIOPHARMACEUTICAL INDUSTRY, Eds. Shukla, A.; Etzel, M. and Gadam, S., CRC Press (2006) ISBN 1-57444-517-0
   SAITOH T. and HINZE W. L.: "USE OF SURFACTANT-MEDIATED PHASE SEPARATION (CLOUD POINT EXTRACTION) WITH AFFINITY LIGANDS FOR THE EXTRACTION OF HYDROPHILIC PROTEINS", TALANTA, ELMSFORD,NY, US, Bd. 42, Nr. 1, 1. Januar 1995 (1995-01-01), Seiten 119-127
   WO 03/089906 A (UNIV FLORIDA [US]) 30. Oktober 2003 (2003-10-30)

### Bezugszeichenliste:

- 1: Erste Mischkammer
- 2: zweite Mischkammer
- 3: dritte Mischkammer
- 4: vierte Mischkammer
- 5: fünfte Mischkammer
- 6: erster Separator
- 7: zweiter Separator
- 8: erster Hochgradientenmagentseparator
- 9: zweiter Hochgradientenmagentseparator
- 10: Ausgangslösung
- 11: Tensid
- 12: Funktionelle Magnetpartikel
- 13: Wässrige Tensidlösung
- 14: Permanentmagnet
- 15: Abwasserstrom
- 16: Tensidhaltige Phase
- 17: Elutionspuffer
- 18: Gemisch
- 19: Partikelrezyklierung
- 20: Zielsubstanzlösung

## Patentansprüche

1. Verfahren zur magnetisch unterstützen Extraktion einer Zielsubstanz aus einer wässrigen Lösung mit mehreren Substanzen, umfassend folgende Verfahrensschritte:
a) Einmischen von funktionellen magnetischen Partikeln mit einer Affinität zu der Zielsubstanz, wobei die Zielsubstanz sich an die Partikel anbindet, sowie eines Tensids in die wässrige Lösung mit den magnetischen Partikeln, wobei sich eine partikelhaltige einphasige wässrige Tensidlösung ausbildet,
b) Überführung der Tensidlösung in einen zweiphasigen Zustand, wobei eine erste partikel- und tensidhaltige disperse Phase in einer diese umgebenden zweiten Phase entsteht, wobei die tensidhaltige Phase ein geringeres Aufnahmevermögen für ungebundene gelöste Substanzen in der Lösung aufweist als die zweite Phase,
c) Separierung der Phasen im Zweiphasengebiet durch Einwirken eines Magnetfeldes sowie
d) Abtrennung der tensidhaltigen Phase mit den magnetischen Partikeln mit der Zielsubstanz.

2. Verfahren nach Anspruch 1, umfassend die weitern nachfolgenden Verfahrensschritte:
a) Zugabe eines wässrigen Elutionspuffers in die tensidhaltige Phase unter Bildung eines Gemischs,
b) Überführung des Gemischs in einen einphasigen Zustand, wobei die Zielsubstanz von den magnetischen Partikeln gelöst wird,
c) Erneute Überführung der einphasigen Lösung in einen zweiphasigen Zustand, wobei eine Phase die Zielsubstanz und die andere Phase die magnetischen Partikel enthält sowie
d) Separierung der Phasen durch Einwirken eines Magnetfeldes.

3. Verfahren nach Anspruch 2, umfassend eine parallele Einmischung von magnetischen Partikeln und tensidhaltiger Phase in die wässrige Lösung mit der Zielsubstanz.

4. Verfahren nach Anspruch 2, umfassend eine serielle Einmischung von magnetischen Partikeln und anschließend der Tenside in die wässrige Lösung mit der Zielsubstanz.

5. Verfahren nach einem der vorgenannten Ansprüche, **dadurch gekennzeichnet, dass** nach der Abtrennung der ersten tensidhaltigen Phase mit den magnetischen Partikeln mit der Zielsubstanz eine weitere magnetische Abtrennung der magnetischen Partikel mit der Zielsubstanz aus der tensidhaltigen Phase erfolgt.

6. Verfahren nach einem der vorgenannten Ansprüche 1 bis 5, **dadurch gekennzeichnet, dass** die Überführung der Lösung in einen ein- oder zweiphasigen Zustand mittels eines Temperaturwechsels erfolgt.

7. Verfahren nach einem der vorgenannten Ansprüche 1 bis 5, **dadurch gekennzeichnet, dass** die Überführung der Lösung in einen ein- oder zweiphasigen Zustand mittels einer Änderung des pH-Wertes in der Lösung erfolgt.

8. Verfahren nach einem der vorgenannten Ansprüche 1 bis 5, **dadurch gekennzeichnet, dass** die Überführung der Lösung in einen ein- oder zweiphasigen Zustand mittels eines Ionenstärkewechsels erfolgt.

## Claims

1. Method for the magnetically extracting a target substance from an aqueous solution with a plurality of substances, comprising the following method steps:
a) admixing functional magnetic particles having an affinity for the target substance, wherein the target substance bonds to the particles, and of a tenside (surfactant) into the aqueous solution with the magnetic particles, wherein a single-phase aqueous surfactant solution containing the particles is formed,
b) transferring the surfactant solution into a two-phase state, wherein a first disperse phase, containing particles and surfactant, arises in a surrounding second phase, wherein the surfactant-containing phase has a lower absorption capacity for the substances in solution than the second phase,
c) separating the phases in the two-phase region by applying a magnetic field, and
d) removing the surfactant-containing phase comprising the magnetic particles with the target substance.

2. Method according to claim 1, comprising the following further method steps:
a) adding an aqueous elution buffer into the surfactant-containing phase, with the formation of a mixture,
b) transferring the mixture into a single-phase state, wherein the target substance is released from the magnetic particles,
c) repeating transfer of the single-phase solution into a two-phase state, wherein one phase contains the target substance and the other phase contains the magnetic particles, and
d) separating the phases by applying a magnetic field.

3. Method according to claim 2, comprising a parallel admixing of magnetic particles and surfactant-containing phase into the aqueous solution with the target substance.

4. Method according to claim 2, comprising a serial admixing of magnetic particles and then of the surfactants into the aqueous solution with the target substance.

5. Method according to any one of the preceding claims, **characterised in that**, after the separation of the first surfactant-containing phase with the magnetic particles with the target substance, a further magnetic separation of the magnetic particles with the target substance from the surfactant-containing phase takes place.

6. Method according to any one of the preceding claims 1 to 5, **characterised in that** the transfer of the solution into a single-phase or two-phase state takes place by means of a temperature change.

7. Method according to any one of the preceding claims 1 to 5, **characterised in that** the transfer of the solution into a single-phase or two-phase state takes place by means of a change of the pH value in the solution.

8. Method according to any one of the preceding claims 1 to 5, **characterised in that** the transfer of the solution into a single-phase or two-phase state takes place by means of an ion strength change.

## Revendications

1. Procédé d'extraction à assistance magnétique d'une substance cible d'une solution aqueuse renfermant plusieurs substances comportant les étapes suivantes consistant à:
a) mélanger des particules magnétiques fonctionnelles ayant une affinité avec la substance cible, cette substance-cible se liant à ces particules, ainsi qu'un agent tensio-actif renfermé dans la solution aqueuse avec les particules magnétiques, une solution aqueuse tensio-active monophasée renfermant les particules étant formée,
b) transférer la solution tensio-active dans un état biphasé, une première phase renfermant les particules et l'agent tensio-actif étant dispersée dans une seconde phase entourant celle-ci, la phase renfermant l'agent tensio-actif ayant une aptitude de captage des substances dissoutes non liées présentes dans la solution plus faible que la seconde phase,
c) séparer les phases dans la zone biphasée par action d'un champ magnétique, et
d) séparer la phase renfermant l'agent tensioactif et les particules magnétiques liées à la substance-cible.

2. Procédé conforme à la revendication 1,
comprenant les étapes suivantes consistant à :
a) ajouter un tampon d'élution aqueux dans la phase renfermant l'agent tensioactif en formant un mélange,
b) transférer le mélange dans un état monophasé, la substance-cible étant dissoute par les particules magnétiques,
c) transférer à nouveau la solution monophasée dans un état biphasé, une phase renfermant la substance-cible et l'autre phase renfermant les particules magnétiques, et
d) séparer les phases par action d'un champ magnétique.

3. Procédé conforme à la revendication 2,
comprenant un mélange parallèle des particules magnétiques et de la phase renfermant l'agent tensio-actif dans la solution aqueuse renfermant la substance-cible.

4. Procédé conforme à la revendication 2, comprenant un mélange en série de particules magnétiques puis de l'agent tensio-actif dans la solution aqueuse renfermant la substance-cible.

5. Procédé conforme à l'une des revendications précédentes,
**caractérisé en ce qu'**
après la séparation de la première phase renfermant l'agent tensio-actif et les particules magnétiques liées à la substance-cible, une autre séparation magnétique des particules magnétiques liées à la substance-cible de la phase renfermant l'agent tensio-actif est effectuée.

6. Procédé conforme à l'une des revendications précédentes 1 à 5,
**caractérisé en ce que**
le transfert de la solution dans un état monophasé ou biphasé est effectué par modification de la température.

7. Procédé conforme à l'une des revendications précédentes 1 à 5,
**caractérisé en ce que**
le transfert de la solution dans un état monophasé ou biphasé est effectué par modification du pH de la solution.

8. Procédé conforme à l'une des revendications précédentes 1 à 5,
**caractérisé en ce que**
le transfert de la solution dans un état monophasé ou dans un état biphasé s'effectue par modification de la force ionique.
